(19) Europäisches Patentamt / European Patent Office / Office européen des brevets

(11) **EP 2 578 204 B1**

(12) **EUROPEAN PATENT SPECIFICATION**


(45) Date of publication and mention of the grant of the patent:
**12.07.2017 Bulletin 2017/28**

(21) Application number: **11786706.9**

(22) Date of filing: **26.05.2011**

(51) Int Cl.:
***A61K 8/60*** (2006.01) ***A61K 8/06*** (2006.01)
***A61K 8/37*** (2006.01) ***A61Q 1/14*** (2006.01)
***A61Q 19/00*** (2006.01) ***A61K 8/49*** (2006.01)
***A61Q 19/08*** (2006.01) ***A61Q 19/10*** (2006.01)

(86) International application number:
**PCT/JP2011/062064**

(87) International publication number:
**WO 2011/149007 (01.12.2011 Gazette 2011/48)**


(54) **OIL-IN-WATER TYPE EMULSION COMPOSITION**

ÖL-IN-WASSER-EMULSIONSZUSAMMENSETZUNG

COMPOSITION D'ÉMULSION DE TYPE HUILE DANS L'EAU


(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.05.2010 JP 2010121632**

(43) Date of publication of application:
**10.04.2013 Bulletin 2013/15**



(73) Proprietor: **Kao Corporation**
**Chuo-ku**
**Tokyo 103-8210 (JP)**

(72) Inventor: **HAYASE, Motoi**
**Odawara-shi**
**Kanagawa 250-0002 (JP)**

(74) Representative: **Hoffmann Eitle**
**Patent- und Rechtsanwälte PartmbB**
**Arabellastraße 30**
**81925 München (DE)**



(56) References cited:
**EP-A1- 1 964 546** **EP-A1- 2 055 314**
**JP-A- 2009 149 566** **JP-A- 2009 149 566**
**JP-A- 2009 149 567** **JP-A- 2009 167 159**
**JP-A- 2009 167 159** **JP-A- 2011 168 527**
**JP-A- 2011 168 548**

- **DATABASE WPI Week 200951 Thomson Scientific, London, GB; AN 2009-L99013 XP002740641, & JP 2009 167157 A (TOYOBO KK) 30 July 2009 (2009-07-30)**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



EP 2 578 204 B1

## Description

[Field of the Invention]

The present invention relates to an oil-in-water emulsion composition superior in stability and feel.

[Background of the Invention]

Surfactants having an alkylene oxide group such as an ethylene oxide, propylene oxide or butylene oxide group in the molecule have hitherto been widely used in applications including cosmetics and pharmaceuticals. However, their use has been called into question in recent years in terms of safety and environmental burdens. Formulations using surfactants free of alkylene oxide groups such as polyglycerol fatty acid ester and sucrose fatty acid ester are being studied in the development of cosmetics.

Oil-in-water emulsion compositions are superior to water-in-oil emulsion compositions in feel upon application to the skin, and thus are often used as skin compositions for external use and the like. Therefore, oil-in-water emulsion formulations have been studied using such highly hydrophilic surfactants such as polyglycerol fatty acid ester and sucrose fatty acid ester (see Patent Documents 1 to 4).

However, use of such highly hydrophilic surfactants such as polyglycerol fatty acid ester and sucrose fatty acid ester causes not only functional problems such as low water resistance but also stickiness or the like in the coatings, resulting in decreased water resistance and decreased feeling upon use, disadvantageously. This tendency is particularly significant in cases such as cosmetics and pharmaceuticals where such surfactants are present together with other water-soluble active ingredients. Therefore, it is difficult to prepare oil-in-water emulsion compositions having excellent feel without stickiness or the like and having superior water resistance and stability when using highly hydrophilic surfactants such as polyglycerol fatty acid ester and sucrose fatty acid ester.

In recent years, mannosyl erythritol lipids have been developed as one kind of novel biosurfactants, and are known as skin-friendly surfactants (see Patent Document 5, 6 and 7).

[Prior Art List]

[Patent Document]

[Patent Document 1] JP-A-2007-153858
[Patent Document 2] JP-A-11-262653
[Patent Document 3] JP-A-11-71261
[Patent Document 4] JP-A-2008-106043
[Patent Document 5] JP-A-2009-167159
[Patent Document 6] JP-A-2009-149556
[Patent Document 7] JP-A-2009-167157

[Summary of the Invention]

The present invention provides an oil-in-water emulsion composition containing the following components (A) to (C), wherein the combined HLB of the surfactants in the composition is 5 to 7:

(A) an oil containing 78 mass% or more of a fatty acid triglyceride having one or more fatty acid groups each having 10 to 22 carbon atoms,
(B) a sorbitan fatty acid ester having an HLB of 6 or less, and
(C) a mannosyl erythritol lipid.

[Effects of the Invention]

The oil-in-water emulsion composition of the present invention is an oil-in-water emulsion composition stable and superior in a feeling upon use (spreadability, freshness) and can be suitably used for applications such as cosmetics and pharmaceuticals.

[Embodiments for carrying out the Invention]

The mannosyl erythritol lipid described above has an HLB of about 8.7 and is lipophilic to some extent, and thus is expected to provide formulations with water resistance and stability. However, it is difficult to form an oil-in-water emulsion composition with the mannosyl erythritol lipid alone and the mannosyl erythritol lipid has raw material odor, resulting in making it difficult to use it in large amounts, disadvantageously.

Accordingly, the present invention relates to provision of a stable oil-in-water emulsion composition which has an excellent feel upon application and a fresh feeling upon use, using a mannosyl erythritol lipid.

As a result of studies on emulsion systems by combining mannosyl erythritol lipids with various emulsifiers and oils in this context, the present inventor has quite unexpectedly found that, in contrast to the fact that water-in-oil emulsion compositions are typically formed using low-HLB surfactants, use of a mannosyl erythritol lipid in combination with a sorbitan fatty acid ester having an HLB of 6 or less, and 78 mass% or more of a fatty acid triglyceride in an oil, wherein the combined HLB of the surfactants is 5 to 7, provides a stable oil-in-water emulsion composition, and provides an oil-in-water emulsion composition having an excellent feel upon application and an excellent feeling upon use without greasy feel. This finding led to the completion of the present invention.

Preferred embodiments of the oil-in-water emulsion composition of the present invention are as follows, for example.

[1] An oil-in-water emulsion composition containing the following components (A) to (C), wherein the combined HLB of the surfactants in the composition is 5 to 7:

(A) an oil containing 78 mass% or more of a fatty acid triglyceride having one or more fatty acid groups each having 10 to 22 carbon atoms,
(B) a sorbitan fatty acid ester having an HLB of 6 or less, and
(C) a mannosyl erythritol lipid.

[2] The oil-in-water emulsion composition according to [1], wherein the content of the component (A) is 1 to 80 mass%.
[3] The oil-in-water emulsion composition according to [1] or [2], wherein the content of the component (A) is 2 to 60 mass%.
[4] The oil-in-water emulsion composition according to any one of [1] to [3], wherein the content of the component (A) is 5 to 50 mass%.
[5] The oil-in-water emulsion composition according to any one of [1] to [4], wherein the content of the component (A) is 5 to 40 mass%.
[6] The oil-in-water emulsion composition according to any one of [1] to [5], wherein the content of the component (B) is 0.01 to 20 mass%.
[7] The oil-in-water emulsion composition according to any one of [1] to [6], wherein the content of the component (B) is 0.1 to 12 mass%.
[8] The oil-in-water emulsion composition according to any one of [1] to [7], wherein the content of the component (B) is 1 to 10 mass%.
[9] The oil-in-water emulsion composition according to any one of [1] to [8], wherein the content of the component (C) is 0.005 to 8 mass%.
[10] The oil-in-water emulsion composition according to any one of [1] to [9], wherein the content of the component (C) is 0.01 to 3 mass%.
[11] The oil-in-water emulsion composition according to any one of [1] to [10], wherein the content of the component (C) is 0.02 to 2 mass%.
[12] The oil-in-water emulsion composition according to any one of [1] to [11], wherein the content of the fatty acid triglyceride having one or more fatty acid groups each having 10 to 22 carbon atoms in the component (A) is 80 to 100 mass%.
[13] The oil-in-water emulsion composition according to any one of [1] to [12], wherein the content of the fatty acid triglyceride having one or more fatty acid groups each having 10 to 22 carbon atoms in the component (A) is 88 to 100 mass%.
[14] The oil-in-water emulsion composition according to any one of [1] to [13], wherein the component (B) is a sorbitan fatty acid ester having an HLB of 1 to 6 and having a linear or branched fatty acid group having 12 to 22 carbon atoms.
[15] The oil-in-water emulsion composition according to any one of [1] to [14], wherein the component (B) is a sorbitan fatty acid ester having an HLB of 3 to 5.5 and having a branched fatty acid group having 15 to 20 carbon atoms.
[16] The oil-in-water emulsion composition according to any one of [1] to [15], wherein the component (B) is one or more selected from the group consisting of sorbitan monostearate, sorbitan monoisostearate, sorbitan sesquiisostearate, sorbitan sesquioleate, sorbitan monooleate and sorbitan trioleate.

[17] The oil-in-water emulsion composition according to any one of [1] to [16], wherein the component (B) is one or more selected from the group consisting of sorbitan monoisostearate, sorbitan sesquiisostearate, sorbitan sesquioleate and sorbitan monooleate.

[18] The oil-in-water emulsion composition according to any one of [1] to [17], wherein the fatty acid triglyceride is a fatty acid triglyceride having two or more fatty acid groups each having 10 to 22 carbon atoms.

[19] The oil-in-water emulsion composition according to any one of [1] to [18], wherein the fatty acid triglyceride is a fatty acid triglyceride having three fatty acid groups each having 10 to 22 carbon atoms.

[20] The oil-in-water emulsion composition according to any one of [1] to [19], wherein the fatty acid triglyceride is one or more selected from the group consisting of olive oil, macadamia nut oil, meadowfoam oil, castor oil, safflower oil, sunflower oil, avocado oil, canola oil, apricot kernel oil, rice germ oil, rice bran oil, glyceryl triacetyl hydroxystearate, glyceryl triacetyl ricinoleate, triisopalmitic acid glyceride, glyceryl triisostearate, glyceryl triundecanoate, glyceryl trihydroxystearate, glyceryl trioleate, glyceryl tri(caprylate/caprate/isostearate/adipate), glyceryl tri(caprylate/caprate), glyceryl tri(caprylate/caprate/myristate/stearate), glyceryl tri(caprylate/caprate/laurate), glyceryl tri(caprylate/caprate/linoleate), glyceryl tricaprate, glyceryl tritallowate, glyceryl tri(tallowate/mustelate/morrhuate), trimyristic acid glyceride, glyceryl tristearate, glyceryl tripalmitate, glyceryl tri-2-heptylundecanoate, glyceryl tribehenate, glyceryl trimyristate, glyceryl tri(mustelate/palmitate), glyceryl tricocoate, glyceryl trilaurate, glyceryl trilanolate, glyceryl tri(ricinoleate/caproate/caprylate/caprate) and glyceryl trilinoleate.

[21] The oil-in-water emulsion composition according to any one of [1] to [20], wherein the fatty acid triglyceride is one or more selected from the group consisting of olive oil, macadamia nut oil, glyceryl tri(caprylate/caprate), glyceryl triisostearate, glyceryl tripalmitate, glyceryl tri-2-heptylundecanoate, glyceryl tribehenate, glyceryl trimyristate, glyceryl tricocoate and glyceryl trilaurate.

[22] The oil-in-water emulsion composition according to any one of [1] to [21], wherein the component (C) is one or more selected from the group consisting of MEL-A, MEL-B, MEL-C and MEL-D.

[23] The oil-in-water emulsion composition according to any one of [1] to [22], , wherein the surfactants have a combined HLB of 5 to 7.

[24] The oil-in-water emulsion composition according to any one of [1] to [23], which is substantially free of a surfactant having an HLB of 10 or more.

[25] The oil-in-water emulsion composition according to any one of [1] to [24], which is substantially free of a surfactant having a polyalkylene oxide group.

[26] The oil-in-water emulsion composition according to any one of [1] to [25], containing 5 to 50 mass% of (A) an oil containing 80 mass% or more of a fatty acid triglyceride having three fatty acid groups each having 10 to 22 carbon atoms.

[27] The oil-in-water emulsion composition according to any one of [1] to [26], containing 0.1 to 12 mass% of (B) a sorbitan fatty acid ester having an HLB of 3 to 5.5 and having a branched fatty acid group having 15 to 20 carbon atoms.

[28] The oil-in-water emulsion composition according to any one of [1] to [27], containing 0.01 to 3 mass% of (C) a mannosyl erythritol lipid which is one or more selected from the group consisting of MEL-A, MEL-B, MEL-C and MEL-D.

[29] The oil-in-water emulsion composition according to any one of [1] to [28], containing 5 to 50 mass% of (A) an oil containing 80 mass% or more of a fatty acid triglyceride having three fatty acid groups each having 10 to 22 carbon atoms, and containing 0.1 to 12 mass% of (B) a sorbitan fatty acid ester having an HLB of 3 to 5.5 and having a branched fatty acid group having 15 to 20 carbon atoms.

[30] The oil-in-water emulsion composition according to any one of [1] to [29], containing 5 to 50 mass% of (A) an oil containing 80 mass% or more of a fatty acid triglyceride having three fatty acid groups each having 10 to 22 carbon atoms, and containing 0.01 to 3 mass% of (C) a mannosyl erythritol lipid which is one or more selected from the group consisting of MEL-A, MEL-B, MEL-C and MEL-D.

[31] The oil-in-water emulsion composition according to any one of [1] to [30], containing 0.1 to 12 mass% of (B) a sorbitan fatty acid ester having an HLB of 3 to 5.5 and having a branched fatty acid group having 15 to 20 carbon atoms, and containing 0.01 to 3 mass% of (C) a mannosyl erythritol lipid which is one or more selected from the group consisting of MEL-A, MEL-B, MEL-C and MEL-D.

The oil (A) used in the oil-in-water emulsion composition of the present invention contains 78 mass% or more of a fatty acid triglyceride having one or more fatty acid groups each having 10 to 22 carbon atoms. The fatty acid triglyceride is preferably a fatty acid triglyceride having two or more fatty acid groups each having 10 to 22 carbon atoms, particularly preferably a fatty acid triglyceride having three fatty acid groups each having 10 to 22 carbon atoms, in terms of a feeling upon use, in particular, lightness and freshness when the resulting oil-in-water emulsion system is applied

to the skin. Examples of such fatty acid triglycerides include vegetable oils such as olive oil, macadamia nut oil, meadowfoam oil, castor oil, safflower oil, sunflower oil, avocado oil, canola oil, apricot kernel oil, rice germ oil and rice bran oil; and synthetic glycerides such as glyceryl triacetyl hydroxystearate, glyceryl triacetyl ricinoleate, triisopalmitic acid glyceride, glyceryl triisostearate, glyceryl triundecanoate, glyceryl trihydroxystearate, glyceryl trioleate, glyceryl tri(caprylate/caprate/isostearate/adipate), glyceryl tri(caprylate/caprate), glyceryl tri(caprylate/caprate/myristate/stearate), glyceryl tri(caprylate/caprate/laurate), glyceryl tri(caprylate/caprate/linoleate), glyceryl tricaprate, glyceryl tritallowate, glyceryl tri(tallowate/mustelate/morrhuate), trimyristic acid glyceride, glyceryl tristearate, glyceryl tripalmitate, glyceryl tri-2-heptylundecanoate, glyceryl tribehenate, glyceryl trimyristate, glyceryl tri(mustelate/palmitate), glyceryl tricocoate, glyceryl trilaurate, glyceryl trilanolate, glyceryl tri(ricinoleate/caproate/caprylate/caprate) and glyceryl trilinoleate. Those commercially available as raw materials for cosmetics and pharmaceuticals can be used. In the present invention, one or more of them can be selected and used.

Among these, olive oil, macadamia nut oil, glyceryl tri(caprylate/caprate), glyceryl triisostearate, glyceryl tripalmitate, glyceryl tri-2-heptylundecanoate, glyceryl tribehenate, glyceryl trimyristate, glyceryl tricocoate, glyceryl trilaurate and the like are preferred to form an oil-in-water emulsion system and achieve excellent feel upon application and fresh feeling.

The content of the fatty acid triglyceride in the oil (A) is importantly 78 mass% or more, more preferably 80 to 100 mass%, even more preferably 85 to 100 mass%, rticularly preferably 88 to 100 mass%, to provide a stable oil-in-water emulsion system and achieve excellent feel upon application.

Oily components other than the fatty acid triglyceride which may be contained in the oil (A) include squalanes such as olive squalane, rice squalane and shark squalane; silicone oils such as dimethylpolysiloxane and cyclic silicone; hydrocarbons such as paraffin, liquid paraffin, vaseline, and olefin oligomers; vegetable oils such as jojoba oil; triglycerides having a fatty acid group having eight or less carbon atoms such as glyceryl tri-2-ethylhexanoate; waxes such as yellow beeswax, Japan wax and carnauba wax; ester oils such as octyldodecyl myristate, cetyl palmitate, isostearyl isostearate and isopropyl myristate; higher alcohols such as cetanol, behenyl alcohol, isostearyl alcohol, jojoba alcohol, oleyl alcohol, stearyl alcohol and branched long-chain aliphatic alcohol; sterols and derivatives thereof such as cholesterol, phytosterol, branched fatty acid cholesterol ester and macadamia nut oil fatty acid phytosteryl ester; processed oils such as hydrogenated oils; higher fatty acids such as stearic acid, myristic acid, long-chain iso fatty acid and long-chain anteiso fatty acid; ethers such as dicapryl ether; and terpenes such as limonene and hydrogenated bisabolol.

The content of the component (A) in the oil-in-water emulsion composition of the present invention is preferably 1 to 80 mass%, more preferably 2 to 60 mass%, even more preferably 5 to 50 mass%, particularly preferably 5 to 40 mass%, to provide a stable oil-in-water emulsion system and in terms of oiliness.

In the present invention, a stable oil-in-water emulsion composition can be obtained using (B) a sorbitan fatty acid ester having an HLB of 6 or less in addition to (C) a mannosyl erythritol lipid which is one kind of a biosurfactant.

The HLB value is an index indicating the ratio of relative affinities of a surfactant for both liquids in an oil-water system, and refers to an HLB value at 25°C as defined by Griffin. The HLB value by Griffin is defined in J. Soc. Cosm. Chem., 1954, 5:249-256.

Generally, surfactants having a low HLB value (particularly an HLB of about 3 to 6) are highly lipophilic and tend to produce water-in-oil emulsion compositions. Surfactants having a large HLB value (particularly an HLB of about 8 to 18) are highly hydrophilic and tend to produce oil-in-water emulsion compositions.

A sorbitan fatty acid ester having an HLB of 6 or less which is used in the oil-in-water emulsion compositions of the present invention is generally used as emulsifiers for water-in-oil emulsion composition due to their low HLB values. However, quite unexpectedly, stable oil-in-water emulsion compositions can be obtained using the sorbitan fatty acid ester in combination with (C) mannosyl erythritol lipids.

Examples of the sorbitan fatty acid ester having an HLB of 6 or less according to the present invention include sorbitan monostearate (HLB = 4.7), sorbitan monoisostearate (HLB = 5), sorbitan sesquiisostearate (HLB = 4.5), sorbitan sesquioleate (HLB = 3.7), sorbitan monooleate (HLB = 4.7) and sorbitan trioleate (HLB = 1.7).

Among such sorbitan fatty acid esters, sorbitan fatty acid esters having an HLB of 1 to 6 and having a linear or branched fatty acid group having 12 to 22 carbon atoms are preferred, and sorbitan fatty acid esters having an HLB of 3 to 5.5 and having a branched fatty acid group having 15 to 20 carbon atoms are more preferred to provide stable oil-in-water emulsion systems with mannosyl erythritol lipids. Preferred examples include sorbitan monoisostearate (HLB = 5), sorbitan sesquiisostearate (HLB = 4.5), sorbitan sesquioleate (HLB = 3.7) and sorbitan monooleate (HLB 4.7).

The content of the component (B) in the oil-in-water emulsion composition of the present invention is preferably 0.01 to 20 mass%, more preferably 0.1 to 15 mass%, even more preferably 0.1 to 12 mass%, particularly preferably 1 to 10 mass%, to provide a stable oil-in-water emulsion system and in terms of irritation.

The oil-in-water emulsion composition of the present invention is desirably substantially free of a surfactant having a polyalkylene oxide group in terms of safety and environmental burden, because a stable oil-in-water emulsion system can be provided even if the composition does not contain a surfactant having a polyalkylene oxide group.

The mannosyl erythritol lipid (C) used in the present invention (hereinafter also called MEL) is a natural surfactant made by yeast, and is a compound which may have a structure in which the 1-position of mannose is substituted with erythritol, the 2- and 3-positions of mannose are substituted with acyl groups and the 4- and 6-positions of mannose optionally are substituted with acetyl groups.

Four known MELs based on the presence or absence of acetyl groups at the 4- and 6-positions of mannose are MEL-A, MEL-B, MEL-C and MEL-D. The following formula (I) shows the structure of MEL-A. In the formula (I), $R^1$ and $R^2$ represent hydrocarbon groups. Specifically, MEL-A is a compound of the formula (I) having acyl groups each having 5 to 19 carbon atoms at the 2- and 3-positions of mannose, and having acetyl groups at the 4- and 6-positions of mannose. MEL-B is a compound of the formula (I) where the acetyl group ($CH_3CO$) at the 4-position of mannose is replaced with H. MEL-C is a compound of the formula (I) where the acetyl group ($CH_3CO$) at the 6-position of mannose is replaced with H. MEL-D is a compound of the formula (I) where the acetyl groups ($CH_3CO$) at the 4- and 6-positions of mannose are both replaced with H.

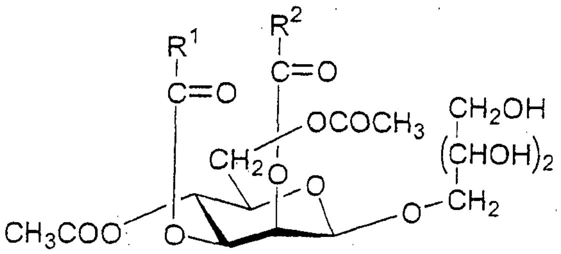

(I)

MEL-A

[$R^1$ and $R^2$ are the same or different and each represent a hydrogen atom, a linear or branched alkyl group having 1 to 18 carbon atoms, or a linear or branched alkenyl, alkadienyl or alkatrienyl group having 2 to 18 carbon atoms.]

In accordance with the formula (I), MEL is a compound having saturated or unsaturated, linear or branched acyl groups each having 1 to 19 carbon atoms at the 2- and 3-positions of mannose, and having acetyl groups at the 4- and 6-positions of mannose (either one or both of acetyl groups at the 4- and 6-positions of mannose may be a hydroxyl group(s)). The total number of carbon atoms in the acyl groups at the 2- and 3-positions is preferably 10 to 38, more preferably 14 to 30.

Preferred groups for $R^1$ and $R^2$ include $C_7$-$C_{11}$ alkyl groups such as $CH_3(CH_2)_6$, $CH_3(CH_2)_8$ and $CH_3(CH_2)_{10}$; $C_7$-$C_{11}$ alkenyl groups such as $C_7H_{14}$, $C_9H_{18}$ and $C_{11}H_{22}$; $C_7$-$C_{11}$ alkadienyl groups such as $C_7H_{12}$, $C_9H_{16}$ and $C_{11}H_{20}$; and $C_7$-$C_{11}$ alkatrienyl groups such as $C_7H_{10}$, $C_9H_{14}$ and $C_{11}H_{18}$.

Commercially available products such as SurfMellow B (manufactured by Toyobo Co., Ltd.) may be used as such MELs.

The content of the component (C) in the oil-in-water emulsion composition of the present invention is preferably 0.005 to 8 mass%, more preferably 0.01 to 5 mass%, even more preferably 0.01 to 3 mass%, particularly preferably 0.02 to 3 mass%. Mannosyl erythritol lipids are expensive and also may generate raw material odor, because they are produced by fermenting vegetable oils, vegetable proteins or the like. If the content is within this range, a stable oil-in-water emulsion system is provided, usability is high, and raw material odor is slight, favorably.

In the oil-in-water emulsion compositions of the present invention, when surfactants other than the components (B) and (C) are used in combination, the combined HLB of the surfactants is 5 to 7 in terms of freshness and spreadability. In the present invention, the composition may be substantially free of a surfactant having an HLB of 10 or more, because a stable oil-in-water emulsion system excellent in a feeling upon use can be provided even if the composition does not contain a surfactant having high hydrophilicity, in particular, having an HLB of 10 or more.

The HLB of a surfactant mixture composed of two or more surfactants is determined as follows. The HLB of the surfactant mixture is obtained by arithmetic mean of the HLB values of the respective surfactants based on the blending ratio.

$$\text{Combined HLB} = \Sigma(\text{HLBx} \times \text{Wx}) / \Sigma\text{Wx}$$

HLBx represents the HLB value of a surfactant X.
Wx represents the mass (g) of the surfactant X having the HLBx value.

Components other than the above components in the oil-in-water emulsion composition of the present invention include, but are not limited to, water; color pigments such as tar pigments and iron oxide; preservatives such as paraben and phenoxyethanol; anionic surfactants such as sodium cetyl sulfate and N-stearoyl-L-glutamate; nonionic surfactants such as polyol fatty acid ester, polyglycerol fatty acid ester, modified silicone and sucrose ester; cationic surfactants such as tetraalkylammonium salt; amphoteric surfactants such as betaine, sulfobetaine and sulfoamino acid surfactants; natural surfactants such as lecithin,
lysophosphatidylcholine, ceramide and cerebroside; pigments such as titanium oxide and zinc oxide; antioxidants such as dibutylhydroxytoluene; inorganic salts such as sodium chloride, magnesium chloride, sodium sulfate and potassium nitrate; organic acid salts such as sodium citrate, potassium acetate, sodium succinate, sodium aspartate, sodium lactate, carnitine salt, γ-aminobutyric acid and lipoic acid; chelators such as salts such as ethanolamine hydrochloride, ammonium nitrate and arginine hydrochloride, and edetic acid; neutralizers such as potassium hydroxide, diisopropanolamine and triethanolamine; biopolymers such as hyaluronic acid and collagen; placenta extract; UV absorbers such as hydroxymethoxybenzophenone sulfonate; vitamin A and derivatives thereof such as retinol, retinol acetate and retinol palmitate; vitamin E and derivatives thereof such as α-tocopherol, γ-tocopherol, δ-tocopherol, tocopherol nicotinate and tocopherol acetate; oil-soluble vitamin C derivatives such as ascorbyl palmitate, ascorbyl stearate and ascorbyl tetraisostearate; water-soluble polymers such as polysaccharides extracted from xanthan gum, β-glucan, oat, white jelly fungus and the like, ones extracted from seaweed, like carrageenan, alginic acid and agar, carboxyvinyl polymers, pectin, and alkyl-modified carboxyvinyl polymers; and polyols such as dipropylene glycol, 1,3-butylene glycol, glycerol, propylene glycol, sorbitol, maltitol, diglycerol, raffinose and hexylene glycol.

Plant, seaweed or bacterial extracts may be used for the oil-in-water emulsion compositions of the present invention, and are, for example, extracts extracted from entire plant, leaves, stems, roots, fruits, seeds, flowers, fruit bodies and bacterial cells of artichoke, indigo plant, arnica, aloe, althea, Angelica keiskei, acerola, apricot, almond, Hydrangea macrophylla var thunbergii, chocolate vine, anise, avocado, Artemisia capillaris flower, nettle, strawberry, fennel, turmeric, opuntia, oolong tea, common mallow, rosa multiflora, Echinacea angustifolia, oat, Isodon japonicus, edelweiss, watercress, Phellodendron amurense, Scutellaria baicalensis, Coptis japonica, giant crape myrtle, Hypericum erectum, orange, okra, olive leaf, black currant, Japanese valerian, persimmon, Pyracantha fortuneana, camomile, camu camu, carotte, Artemisia capillaris, Avena fatua, licorice, cucumber, apricot kernel, kiwi, cinchona, quillai, Hedera rhombea, gaba tea, raspberry, shrubby sophora, Sasa veitchii, mulberry, walnut, grapefruit, Geranium thunbergii, gentian, shell ginger, kothalahimbutu, burdock, comfrey, wheat germ, cherry blossom, common soapwort, salvia, Japanese hawthorn, Asarum sieboldii, umbilical cord, Gardenia fruit, meadowsweet, Houttuynia cordata, Spanish flag, sweet flag, ginger, Lithospermum purpurocaeruleum, perilla, white birch, peony, Rehmannia glutinosa, Citrus depressa, simon batatas, field horsetail, star fruit, high mallow, cnidii rhizoma, hawthorn, English ivy, pear, white willow, sage, Swertia japonica, soybean, bitter orange, thyme, Citrus tachibana, garden thyme, tamarind, tea, clove, citrus unshiu peel, camellia, Houttuynia cordata, Angelica acutiloba, peach kernel, bitter orange peel, tomato, garden marigold, Ampelopsis grossedentata, Potentilla erecta, corn, neem, horehound, elder, garlic, carrot, wild rose, Myristica argentea, hibiscus, parsley, banana, rose, Job's tears, lupine, pecan nuts, Japanese cypress, hyssop, Himalayan raspberry, herb Robert, sandalwood, bilberry, loquat, prune, grape, bunchflower daffodil, butterfly bush, rose apple, peppermint, safflower, sponge gourd, ribwort plantain, white genepi, Magnolia obovata, linden, Peucedanum japonicum, peony, hop, jojoba, quince, Rosa rugosa, horse-chestnut, pine, orange, soapberry, melissa, common evening primrose, peach, maple, cornflower, saxifrage, eucalyptus, lily, yuzu, coix seed, mugwort, orchid, lime, lavender lettuce, apple, Artemisia campestris, rooibos, milk vetch, lemon, lemon balm, rose hip, rosemary, green algae, red algae, brown algae, Poria sclerotium, shiitake mushroom, Hypholoma sublateritium, polypore, snow fungus, Ganoderma lucidum, plant worm, yeast, lactic acid bacteria and root nodule bacteria with water, organic solvents such as glycerol, propylene glycol, ethanol and butylene glycol, or mixtures thereof, or oils such as olive oil and macadamia nut oil. Chlorophylls are also plant extracts. Also, culture solutions of lactic acid bacteria or yeast using raw milk, fruit juice, synthetic media or semisynthetic media may be used regardless of removal of the bacterial cells.

The compositions of the present invention are oil-in-water emulsion compositions, and are superior in feel upon application to the skin, for example, spreadability, and excellent in feel such as freshness. Accordingly, the oil-in-water emulsion compositions of the present invention may be used for applications such as cosmetics and pharmaceuticals, and may be suitably applied as external skin compositions such as moisturizing, anti-aging, whitening, pack, massage, sunscreen, makeup base, foundation, lip cream and cleansing formulations.

[Examples]

The present invention will be described in detail below based on examples and comparative examples; however, the present invention is not limited thereto. First, the stability over time and sensory test used in examples and comparative examples will be described below.

[Sensory test]

Samples were used by 20 adult women evaluation panelists, and the evaluation was performed after use based on the number of panelists who answered that the samples gave a smooth feel upon application (spreadability) and no greasy feel after application (freshness). The cleansing and massage formulations were evaluated only for greasy feel after application. The presence or absence of raw material odor was evaluated based on the number of panelists who answered that they feel raw material odor during use.

Examples 1 to 12, Comparative Examples 1 to 24

Essences were prepared with the compositions described in Tables 1 to 3, and the above sensory test was performed.

[Table 1]

| Component | Example | | | | | | | | | | | | | | | | | | |
|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 |
| Olive oil*2 | 20 | | | | 20 | 18 | 17 | 16 | 5 | 40 | 50 | 2 | 50 | 20 | 20 | 20 | 20 | 20 | 20 |
| Macadamia nut oil | | 20 | | | | | | | | | | | | | | | | | |
| Glyceryl tri(caprylate/caprate) | | | 20 | | | | | | | | | | | | | | | | |
| Glyceryl triisostearate | | | | 20 | | | | | | | | | | | | | | | |
| Liquid paraffin | | | | | | 2 | 3 | 4 | | | | | | | | | | | |
| Squalane | | | | | | | | | | | | | | | | | | | |
| Methylphenylpolysiloxane | | | | | | | | | | | | | | | | | | | |
| Jojoba oil | | | | | | | | | | | | | | | | | | | |
| Dimethylpolysiloxane | | | | | | | | | | | | | | | | | | | |
| Olive squalane | | | | | | | | | | | | | | | | | | | |
| Olefin oligomer | | | | | | | | | | | | | | | | | | | |
| Octyldodecyl myristate | | | | | | | | | | | | | | | | | | | |
| Glyceryl tri-2-ethylhexanoate | | | | | | | | | | | | | | | | | | | |
| Isononyl isononanoate | | | | | | | | | | | | | | | | | | | |
| Dioctyl ether | | | | | | | | | | | | | | | | | | | |
| Dioctyl carbonate | | | | | | | | | | | | | | | | | | | |
| Isostearic acid | | | | | | | | | | | | | | | | | | | |
| Jojoba alcohol | | | | | | | | | | | | | | | | | | | |
| Isocetyl myristate | | | | | | | | | | | | | | | | | | | |
| Diisopropyl sebacate | | | | | | | | | | | | | | | | | | | |
| Rice squalane | | | | | | | | | | | | | | | | | | | |
| Sorbitan isostearate [HLB=5.0] | 9 | 9 | 9 | 9 | | 9 | 9 | 9 | 1 | 4.5 | 9 | 0.1 | 8 | 5 | 9 | 1 | 15 | 4.5 | |
| Sorbitan stearate [HLB=4.7] | | | | | 9 | | | | | | | | | | | | | | 5 |
| Mannosyl erythritol lipid *1 | 2 | 2 | 2 | 2 | 2 | 2 | 2 | 2 | 2 | 1 | 2 | 0.04 | 8 | 5 | 0.2 | 1 | 5 | 1 | 2 |

(continued)

| Component | | Example | | | | | | | | | | | | | | | | | | |
|---|---|---|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 | 17 | 18 | 19 |
| POE hydrogenated castor oil (60E.O.) [HLB=14] | | | | | | | | | | | | | | | | | | | | |
| Stearic acid | | | | | | | | | | | | | | | | | | | | 3 |
| Triethanolamine | | | | | | | | | | | | | | | | | | | | 1 |
| Cetyl dimethicone copolyol [HLB=5]*3 | | | | | | | | | | | | | | | | | | | | |
| Pure water | | 69.0 | 69.0 | 69.0 | 69.0 | 69.0 | 69.0 | 69.0 | 84.0 | 92.0 | 54.5 | 39.0 | 97.9 | 34.0 | 70.0 | 70.8 | 78.0 | 60.0 | 74.5 | 69.0 |
| | | | | | | | | | | | | | | | | | | | | |
| Test results | Emulsion type | O/W | O/W | O/W | O/W | O/W | O/W | O/W | O/W | O/W | O/W | O/W | O/W | O/W | O/W | O/W | O/W | O/W | O/W | O/W |
| | Smooth feel upon application | 18 | 19 | 19 | 18 | 19 | 17 | 18 | 18 | 19 | 17 | 19 | 20 | 17 | 17 | 17 | 18 | 15 | 20 | 10 |
| | No greasy feel | 18 | 18 | 20 | 17 | 18 | 18 | 16 | 16 | 18 | 17 | 14 | 19 | 14 | 20 | 19 | 18 | 14 | 20 | 13 |
| | Presence or absence of raw material odor | 2 | 2 | 1 | 1 | 2 | 2 | 2 | 1 | 2 | 2 | 0 | 0 | 6 | 4 | 0 | 1 | 4 | 0 | 3 |

*1 SurfMellow B BG manufactured by Toyobo Co., Ltd. (50% 1,3-butylene glycol solution)
*2 Cropure OL manufactured by Croda Japan KK
*3 ABIL EM90 manufactured by Evonik Industries AG

[Table 2]

| Component | Comparative Example | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| Olive oil*2 | | | | | | | | | | | | |
| Macadamia nut oil | | | | | | | | | | | | |
| Glyceryl tri(caprylate/caprate) | | | | | | | | | | | | |
| Glyceryl triisostearate | | | | | | | | | | | | |
| Liquid paraffin | 20 | | | | | | | | | | | |
| Squalane | | 20 | | | | | | | | | | |
| Methylphenylpolysiloxane | | | 20 | | | | | | | | | |
| Jojoba oil | | | | 20 | | | | | | | | |
| Dimethylpolysiloxane | | | | | 20 | | | | | | | |
| Olive squalane | | | | | | 20 | | | | | | |
| Olefin oligomer | | | | | | | 20 | | | | | |
| Octyldodecyl myristate | | | | | | | | 20 | | | | |
| Glyceryl tri-2-ethylhexanoate | | | | | | | | | 20 | | | |
| Isononyl isononanoate | | | | | | | | | | 20 | | |
| Dioctyl ether | | | | | | | | | | | 20 | |
| Dioctyl carbonate | | | | | | | | | | | | 20 |
| Isostearic acid | | | | | | | | | | | | |
| Jojoba alcohol | | | | | | | | | | | | |
| Isocetyl myristate | | | | | | | | | | | | |
| Diisopropyl sebacate | | | | | | | | | | | | |
| Rice squalane | | | | | | | | | | | | |
| Sorbitan isostearate [HLB=5.0] | 9 | 9 | 9 | 9 | 9 | 9 | 9 | 9 | 9 | 9 | 9 | 9 |
| Sorbitan stearate [HLB=4.7] | | | | | | | | | | | | |
| Mannosyl erythritol lipid *1 | 2 | 2 | 2 | 2 | 2 | 2 | 2 | 2 | 2 | 2 | 2 | 2 |

(continued)

| Component | | Comparative Example | | | | | | | | | | | |
|---|---|---|---|---|---|---|---|---|---|---|---|---|---|
| | | 1 | 2 | 3 | 4 | 5 | 6 | 7 | 8 | 9 | 10 | 11 | 12 |
| POE hydrogenated castor oil (60E.O.) [HLB=14] | | | | | | | | | | | | | |
| Cetyl dimethicone copolyol [HLB=5]*3 | | | | | | | | | | | | | |
| Pure water | | 69.0 | 69.0 | 69.0 | 69.0 | 69.0 | 69.0 | 69.0 | 69.0 | 69.0 | 69.0 | 69.0 | 69.0 |
| | | | | | | | | | | | | | |
| Test results | Emulsion type | W/O | W/O | W/O | W/O | W/O | W/O | W/O | W/O | W/O | W/O | W/O | W/O |
| | Smooth feel upon application | 0 | 0 | 0 | 0 | 0 | 0 | 0 | 0 | 0 | 0 | 0 | 0 |
| | No greasy feel | 0 | 0 | 4 | 0 | 0 | 0 | 0 | 0 | 5 | 8 | 9 | 9 |
| | Presence or absence of raw material odor | 1 | 2 | 2 | 3 | 1 | 2 | 1 | 1 | 1 | 2 | 3 | 3 |

*1 SurfMellow B BG manufactured by Toyobo Co., Ltd. (50% 1,3-butylene glycol solution)
*2 Cropure OL manufactured by Croda Japan KK
*3 ABIL EM90 manufactured by Evonik Industries AG

[Table 3]

| Component | Comparative Example | | | | | | | | | | | | |
|---|---|---|---|---|---|---|---|---|---|---|---|---|---|
| | 13 | 14 | 15 | 16 | 17 | 18 | 19 | 20 | 21 | 22 | 23 | 24 | 25 |
| Olive oil*2 | | | | | | 20 | 20 | 20 | 20 | | 15 | 14 | 10 |
| Macadamia nut oil | | | | | | | | | | | | | |
| Glyceryl tri(caprylate/caprate) | | | | | | | | | | | | | |
| Glyceryl triisostearate | | | | | | | | | | | | | |
| Liquid paraffin | | | | | | | | | | 20 | 5 | | 10 |
| Squalane | | | | | | | | | | | | | |
| Methylphenylpolysiloxane | | | | | | | | | | | | 6 | |
| Jojoba oil | | | | | | | | | | | | | |
| Dimethylpolysiloxane | | | | | | | | | | | | | |
| Olive squalane | | | | | | | | | | | | | |
| Olefin oligomer | | | | | | | | | | | | | |
| Octyldodecyl myristate | | | | | | | | | | | | | |
| Glyceryl tri-2-ethylhexanoate | | | | | | | | | | | | | |
| Isononyl isononanoate | | | | | | | | | | | | | |
| Dioctyl ether | | | | | | | | | | | | | |
| Dioctyl carbonate | | | | | | | | | | | | | |
| Isostearic acid | 20 | | | | | | | | | | | | |
| Jojoba alcohol | | 20 | | | | | | | | | | | |
| Isocetyl myristate | | | 20 | | | | | | | | | | |
| Diisopropyl sebacate | | | | 20 | | | | | | | | | |
| Rice squalane | | | | | 20 | | | | | | | | |
| Sorbitan isostearate [HLB=5.0] | 9 | 9 | 9 | 9 | 9 | 9 | 9 | | | | 9 | 9 | 9 |
| Sorbitan stearate [HLB=4.7] | | | | | | | | | | 5 | | | |
| Mannosyl erythritol lipid *1 | 2 | 2 | 2 | 2 | 2 | | | 2 | 2 | 2 | 2 | 2 | 2 |

(continued)

| Component | | Comparative Example | | | | | | | | | | | | |
|---|---|---|---|---|---|---|---|---|---|---|---|---|---|---|
| | | 13 | 14 | 15 | 16 | 17 | 18 | 19 | 20 | 21 | 22 | 23 | 24 | 25 |
| POE hydrogenated castor oil (60E.O.) [HLB=14] | | | | | | | | 2 | | | | | | |
| Stearic acid | | | | | | | | | | | 3 | | | |
| Triethanolamine | | | | | | | | | | | 1 | | | |
| Cetyl dimethicone copolyol [HLB=5]*3 | | | | | | | | | | 9 | | | | |
| Pure water | | 69.0 | 69.0 | 69.0 | 69.0 | 69.0 | 71.0 | 69.0 | 78.0 | 69.0 | 69.0 | 69.0 | 69.0 | 69.0 |
| | | | | | | | | | | | | | | |
| Test results | Emulsion type | W/O | W/O | W/O | W/O | W/O | W/O | W/O | W/O | W/O | O/W | W/O | W/O | W/O |
| | Smooth feel upon application | 0 | 0 | 0 | 0 | 0 | 0 | 0 | 10 | 0 | 2 | 0 | 0 | 0 |
| | No greasy feel | 0 | 0 | 0 | 0 | 0 | 0 | 0 | 8 | 0 | 0 | 0 | 0 | 0 |
| | Presence or absence of raw material odor | 2 | 3 | 2 | 4 | 2 | 1 | 0 | 2 | 2 | 5 | 2 | 2 | 2 |

*1 SurfMellow B BG manufactured by Toyobo Co., Ltd. (50% 1,3-butylene glycol solution)
*2 Cropure OL manufactured by Croda Japan KK
*3 ABIL EM90 manufactured by Evonik Industries AG

(1) Preparation Method

The respective components were homogeneously mixed and stirred at room temperature.

(2) Properties

The results of the sensory test immediately after the manufacture in examples and comparative examples are shown in Tables 1 to 3. The appearance of the samples was observed by microscopy to determine the emulsion type.

As shown in Tables 1 to 3, in the examples according to the present invention, oil-in-water emulsion compositions were formed and were excellent in a feeling upon use such as spreadability and no greasy feel. On the other hand, in the comparative examples, oil-in-water emulsion compositions were not formed, and feel of the resulting compositions was not satisfactory.

The appearance of the composition of Example 12 was observed after storage at room temperature for half a year to find that the composition was a stable oil-in-water emulsion composition without any change such as separation.

Example 12 (anti-aging essence)

An anti-aging essence was prepared with the composition described in Table 4, and the above sensory test was performed.

[Table 4]

| Anti-aging essence | |
|---|---|
| Component | Content (%) |
| Sorbitan isostearate | 2 |
| Mannosyl erythritol lipid *1 | 0.3 |
| Olive oil *2 | 5 |
| Macadamia nut oil | 1 |
| Jojoba oil | 0.1 |
| Retinol palmitate | 0.1 |
| Ascorbyl tetraisopalmitate | 0.1 |
| Argan oil | 0.1 |
| Dipropylene glycol | 2 |
| Raffinose | 0.5 |
| Ethanol | 5 |
| Phenoxyethanol | 0.2 |
| p-Hydroxybenzoate | 0.3 |
| Camu camu extract | 0.1 |
| Apricot kernel extract | 0.1 |
| N-methyl-L-serine | 0.5 |
| Diisopropylamine dichloroacetate | 0.2 |
| Carrageenan | 0.2 |
| Gigartina tenella extract | 0.1 |
| Bitter orange peel extract | 0.1 |
| Poria sclerotium extract | 0.3 |
| Hibiscus extract | 0.2 |
| Oil-soluble licorice extract | 0.01 |

(continued)

| Anti-aging essence | | |
|---|---|---|
| Component | | Content (%) |
| Avocado extract | | 0.2 |
| Chlorphenesin | | 0.05 |
| Phellodendron amurense extract | | 1 |
| Polyethylene glycol 4000 | | 1 |
| Carboxyvinyl polymer | | 0.2 |
| γ-amino-β-hydroxybutyric acid | | 0.2 |
| Cherry blossom extract | | 0.05 |
| Decaglyceryl diisostearate | | 0.05 |
| Magnesium ascorbate phosphate | | 0.01 |
| Pear juice fermented liquor | | 0.2 |
| Sorbitol solution | | 3 |
| Mulberry bark extract | | 0.1 |
| Edetate | | 0.02 |
| Potassium hydroxide | | 0.05 |
| Oolong tea extract | | 0.1 |
| Flavor | | 0.02 |
| Purified water | | Balance |
| | | |
| Test results | Smooth feel upon application | 18 |
| | No greasy feel | 17 |
| *1 SurfMellow B BG manufactured by Toyobo Co., Ltd. (50% 1,3-butylene glycol solution)<br>*2 Cropure CL manufactured by Croda Japan KK | | |

(1) Preparation Method

The respective components were homogeneously mixed and stirred at room temperature.

(2) Properties

The above properties were tested for the composition of the test example. The results are shown in Table 4. As shown in Table 4, the composition of the example according to the present invention was superior in a feeling upon use.

Example 13 (whitening essence)

A whitening essence was prepared with the composition described in Table 5, and the above sensory test was performed.

[Table 5]

| Whitening essence | |
|---|---|
| Component | Content (%) |
| Sorbitan isostearate | 3 |
| Mannosyl erythritol lipid *1 | 0.1 |

(continued)

| Whitening essence | |
|---|---|
| Component | Content (%) |
| Olive oil *2 | 10 |
| Tocopherol nicotinate | 0.1 |
| Apricot kernel oil | 0.1 |
| Diisopropyl sebacate | 1 |
| Magnolignan *3 | 0.1 |
| Ascorbic acid glucoside | 0.2 |
| Himalayan raspberry extract | 0.1 |
| 1,3-butylene glycol | 5 |
| Xanthan gum | 0.05 |
| Sodium citrate | 0.05 |
| Dipotassium glycyrrhizinate | 0.2 |
| Nicotinamide | 0.1 |
| L-lysine hydrochloride | 0.03 |
| Edetate | 0.02 |
| Sodium polyacrylate | 0.1 |
| Potassium hydroxide | 0.05 |
| Lupine extract | 0.5 |
| Hydrolyzed silk | 0.1 |
| Lactic acid bacteria culture solution | 0.1 |
| Jojoba leaf extract | 0.1 |
| Ethanol | 2 |
| Phenoxyethanol | 0.2 |
| Mevalonolactone | 0.1 |
| Disodium ascorbate sulfate | 0.1 |
| Orchid extract | 0.1 |
| Concentrated glycerine | 2 |
| Rice fermented liquid | 1 |
| White willow extract | 0.2 |
| Common evening primrose extract | 0.2 |
| Pearl protein extract | 0.15 |
| Sodium hyaluronate | 0.001 |
| Pycnogenol | 0.05 |
| Chlorphenesin | 0.05 |
| Flavor | 0.02 |
| Purified water | Balance |
| | |

(continued)

| Whitening essence | | |
|---|---|---|
| Component | | Content (%) |
| Test results | Smooth feel upon application | 20 |
| | No greasy feel | 18 |
| *1 SurfMellow B BG manufactured by Toyobo Co., Ltd. (50% 1,3-butylene glycol solution)<br>*2 Cropure OL manufactured by Croda Japan KK<br>*3 5,5'-Dipropyl-biphenyl-2,2'-diol | | |

(1) Preparation Method

The respective components were homogeneously mixed and stirred at room temperature.

(2) Properties

The above properties were tested for the composition of the test example. The results are shown in Table 5. As shown in Table 5, the composition of the example according to the present invention was superior in a feeling upon use.

Example 14 (moisturizing essence)

A moisturizing essence was prepared with the composition described in Table 6, and the above sensory test was performed.

[Table 6]

| Moisturizing essence | |
|---|---|
| Component | Content (%) |
| Sorbitan isostearate | 3 |
| Mannosyl erythritol lipid *1 | 0.5 |
| Olive oil *2 | 4 |
| Meadowfoam oil | 1 |
| Methylphenylpolysiloxane | 0.5 |
| Retinol palmitate | 0.05 |
| Coenzyme Q10 | 0.03 |
| Olive squalane | 0.2 |
| Field horsetail extract | 0.1 |
| Watercress extract | 0.1 |
| 1, 3-butylene glycol | 5 |
| Concentrated glycerin | 5 |
| $\gamma$-aminobutyric acid | 0.1 |
| Sodium N-stearoyl glutamate | 0.01 |
| Bilberry extract | 0.2 |
| Yeast extract *3 | 1 |
| N-acetylglucosamine | 0.1 |
| Giant crape myrtle extract | 0.1 |
| p-Hydroxybenzoate | 0.2 |

(continued)

| Moisturizing essence | | |
|---|---|---|
| Component | | Content (%) |
| Okra extract | | 0.1 |
| Brown algae extract | | 0.5 |
| Pyracantha fortuneana extract | | 0.1 |
| Aloe extract | | 0.1 |
| Water-soluble licorice extract | | 0.1 |
| POE hydrogenated castor oil (80E.O.) | | 0.05 |
| Chlorphenesin | | 0.05 |
| Surfactin sodium salt | | 0.02 |
| Alkyl-modified carbomer | | 0.03 |
| Hydrolyzed hyaluronic acid | | 0.05 |
| Lactic acid bacteria culture solution | | 0.1 |
| Dipropylene glycol | | 2 |
| Phenoxyethanol | | 0.2 |
| Tremella fuciformis polysaccharide | | 0.01 |
| Gentian extract | | 0.2 |
| Mevalonolactone | | 0.1 |
| Shell ginger leaf extract | | 0.5 |
| Disodium ascorbate sulfate | | 0.1 |
| γ-aminobutyric acid | | 0.2 |
| Edetate | | 0.02 |
| Potassium hydroxide | | 0.05 |
| Yeast extract *4 | | 0.3 |
| Purified water | | Balance |
| | | |
| Test results | Smooth feel upon application | 17 |
| | No greasy feel | 17 |
| *1 SurfMellow B BG manufactured by Toyobo Co., Ltd. (50% 1,3-butylene glycol solution)<br>*2 Cropure CL manufactured by Croda Japan KK<br>*3 TONISKIN manufactured by SILAB<br>*4 DISMUTIN PF manufactured by DSM Nutrition Japan K.K. | | |

(1) Preparation Method

The respective components were homogeneously mixed and stirred at room temperature.

(2) Properties

The above properties were tested for the composition of the test example. The results are shown in Table 6. As shown in Table 6, the composition of the example according to the present invention was superior in a feeling upon use.

Example 15 (cleansing gel)

A cleansing gel was prepared with the composition described in Table 7, and the above sensory test was performed.

[Table 7]

| Cleansing gel | |
|---|---|
| Component | Content (%) |
| Sorbitan isostearate | 2 |
| Mannosyl erythritol lipid *1 | 0.1 |
| Olive oil *2 | 5 |
| Rice bran oil | 10 |
| Jojoba oil | 0.1 |
| Retinol palmitate | 0.1 |
| Ascorbyl tetraisopalmitate | 0.1 |
| Argan oil | 0.1 |
| Dipropylene glycol | 2 |
| Raffinose | 0.5 |
| Ethanol | 5 |
| Phenoxyethanol | 0.2 |
| p-Hydroxybenzoate | 0.3 |
| Rosa multiflora extract | 0.1 |
| Plant worm extract | 0.1 |
| Althea extract | 0.2 |
| Diisopropylamine dichloroacetate | 0.2 |
| POE sorbitan cocoate | 0.05 |
| Star fruit extract | 0.1 |
| Angelica keiskei extract | 0.1 |
| Scutellaria baicalensis extract | 0.3 |
| Job's tears extract | 0.2 |
| Oil-soluble licorice extract | 0.01 |
| Citrus unshiu peel extract | 0.2 |
| Chlorphenesin | 0.05 |
| Houttuynia cordata extract | 1 |
| Bentonite | 0.1 |
| Carboxyvinyl polymer | 0.2 |
| $\gamma$-amino-$\beta$-hydroxybutyric acid | 0.2 |
| Camellia extract | 0.05 |
| Carboxymethylcellulose | 0.02 |
| Magnesium ascorbate phosphate | 0.01 |
| Yuzu extract | 0.2 |
| Sorbitol solution | 3 |

(continued)

| Cleansing gel | | |
|---|---|---|
| Component | | Content (%) |
| Maple leaf extract | | 0.1 |
| Edetate | | 0.02 |
| Potassium hydroxide | | 0.05 |
| Saxifrage extract | | 0.1 |
| Flavor | | 0.02 |
| Purified water | | Balance |
| | | |
| Test results | No greasy feel | 19 |
| *1 SurfMellow B BG manufactured by Toyobo Co., Ltd. (50% 1,3-butylene glycol solution)<br>*2 Cropure OL manufactured by Croda Japan KK | | |

(1) Preparation Method

The respective components were homogeneously mixed and stirred at room temperature.

(2) Properties

The above property was tested for the composition of the test example. The result is shown in Table 7. As shown in Table 7, the composition of the example according to the present invention was superior in a feeling upon use.

Example 16 (massage cream)

A massage cream was prepared with the composition described in Table 8, and the above sensory test was performed.

[Table 8]

| Massage cream | |
|---|---|
| Component | Content (%) |
| Sorbitan isostearate | 4 |
| Mannosyl erythritol lipid *1 | 1 |
| Olive oil *2 | 5 |
| Macadamia nut oil | 1 |
| Avocado oil | 0.5 |
| Rice squalane | 0.1 |
| Triisostearic acid glyceride | 0.1 |
| Macadamia nut oil fatty acid phytosteryl ester | 0.01 |
| Long-chain branched fatty acid cholesteryl ester | 0.01 |
| Argan oil | 0.1 |
| Dipropylene glycol | 2 |
| Raffinose | 0.5 |
| Ethanol | 5 |
| Phenoxyethanol | 0.2 |

(continued)

| Massage cream | | |
|---|---|---|
| Component | | Content (%) |
| p-Hydroxybenzoate | | 0.3 |
| Rehmannia glutinosa extract | | 0.1 |
| Peony extract | | 0.1 |
| N-methyl-L-serine | | 0.5 |
| Diisopropylamine dichloroacetate | | 0.2 |
| Carrageenan | | 0.2 |
| Angelica acutiloba extract | | 0.1 |
| Cnidii rhizoma extract | | 0.1 |
| Citrus depressa extract | | 0.3 |
| Tea extract | | 0.2 |
| Oil-soluble licorice extract | | 0.01 |
| Herb Robert extract | | 0.2 |
| Chlorphenesin | | 0.05 |
| Bilberry leaf extract | | 1 |
| Polyethylene glycol 1000 | | 1 |
| Carboxyvinyl polymer | | 0.2 |
| $\gamma$-amino-$\beta$-hydroxybutyric acid | | 0.2 |
| Artichoke extract | | 0.05 |
| POE hydrogenated castor oil (40E.O.) | | 0.1 |
| Magnesium ascorbate phosphate | | 0.01 |
| Hibiscus fermented liquid | | 0.2 |
| Sorbitol solution | | 3 |
| Common evening primrose extract | | 0.1 |
| Edetate | | 0.02 |
| Potassium hydroxide | | 0.05 |
| Wheat germ extract | | 0.1 |
| Flavor | | 0.02 |
| Purified water | | Balance |
| | | |
| Test results | No greasy feel | 19 |
| *1 SurfMellow B BG manufactured by Toyobo Co., Ltd. (50% 1,3-butylene glycol solution)<br>*2 Cropure OL manufactured by Croda Japan KK | | |

(1) Preparation Method

The respective components were homogeneously mixed and stirred at room temperature.

(2) Properties

The above property was tested for the composition of the test example. The results are shown in Table 8. As shown in Table 8, the composition of the example according to the present invention was superior in a feeling upon use.

## Claims

1. An oil-in-water emulsion composition comprising the following components (A) to (C), wherein the combined HLB of the surfactants in the composition is 5 to 7:

   (A) an oil in which a fatty acid triglyceride having one or more fatty acid groups each having 10 to 22 carbon atoms is contained in an amount of 78 mass% or more in the oil,
   (B) a sorbitan fatty acid ester having an HLB of 6 or less, and
   (C) a mannosyl erythritol lipid.

2. The oil-in-water emulsion composition according to claim 1, wherein the fatty acid triglyceride having one or more fatty acid groups each having 10 to 22 carbon atoms in component (A) is contained in an amount of 80 to 100 mass% in the oil.

3. The oil-in-water emulsion composition according to claim 1 or 2, wherein the content of component (A) is 1 to 80 mass% based on the total amount of the oil-in-water emulsion composition.

4. The oil-in-water emulsion composition according to any one of claims 1 to 3, wherein the content of component (B) is 0.01 to 20 mass% based on the total amount of the oil-in-water emulsion composition.

5. The oil-in-water emulsion composition according to any one of claims 1 to 4, wherein component (B) is a sorbitan fatty acid ester having an HLB of 1 to 6.

6. The oil-in-water emulsion composition according to any one of claims 1 to 5, wherein the content of the component (C) is 0.005 to 8 mass% based on the total amount of the oil-in-water emulsion composition.

7. The oil-in-water emulsion composition according to any one of claims 1 to 6, wherein the fatty acid triglyceride has three fatty acid groups each having 10 to 22 carbon atoms.

8. The oil-in-water emulsion composition according to any one of claims 1 to 7, wherein the fatty acid triglyceride is at least one selected from the group consisting of olive oil, macadamia nut oil, glyceryl tri(caprylate/caprate), glyceryl triisostearate, glyceryl tripalmitate, glyceryl tri-2-heptylundecanoate, glyceryl tribehenate, glyceryl trimyristate, glyceryl tricocoate, and glyceryl trilaurate.

9. The oil-in-water emulsion composition according to claim 1, wherein the components (A), (B) and (C) are as follows:

   (A) an oil in which a fatty acid triglyceride having three fatty acid groups each having 10 to 22 carbon atoms is contained in an amount of 80 to 100 mass% in the oil, the oil is contained in an amount of 5 to 50 mass% based on the total amount of the oil-in-water emulsion composition,
   (B) a sorbitan fatty acid ester having an HLB of 3 to 5.5 and having a branched fatty acid group having 15 to 20 carbon atoms, the sorbitan fatty acid ester is contained in an amount of 0.1 to 12 mass% based on the total amount of oil-in-water emulsion composition and
   (C) 0.01 to 3 mass% of a mannosyl erythritol lipid which is at least one selected from the group consisting of MEL-A, MEL-B, MEL-C and MEL-D.

10. The oil-in-water emulsion composition according to any one of claims 1 to 9, which is substantially free of a surfactant having a polyalkylene oxide group.

11. Use of the oil-in-water emulsion composition according to any one of claims 1 to 10, for cosmetics.

**Patentansprüche**

1. Öl-in-Wasser-Emulsionszusammensetzung, umfassend die nachstehenden Komponenten (A) bis (C), wobei der kombinierte HLB der Tenside in der Zusammensetzung 5 bis 7 beträgt:

    (A) ein Öl, in dem ein Fettsäuretriglycerid mit einer oder mehreren Fettsäuregruppen, die jeweils 10 bis 22 Kohlenstoffatome aufweisen, in einer Menge von 78 Masse-% oder mehr in dem Öl enthalten ist,
    (B) einen Sorbitan-Fettsäureester mit einem HLB von 6 oder weniger, und
    (C) ein Mannosylerythritlipid.

2. Öl-in-Wasser-Emulsionszusammensetzung gemäss Anspruch 1, wobei das Fettsäuretriglycerid mit einer oder mehreren Fettsäuregruppen, die jeweils 10 bis 22 Kohlenstoffatome aufweisen, in der Komponente (A) in einer Menge von 80 bis 100 Masse-% in dem Öl enthalten ist.

3. Öl-in-Wasser-Emulsionszusammensetzung gemäss Anspruch 1 oder 2, wobei der Gehalt der Komponente (A) 1 bis 80 Masse-%, bezogen auf die Gesamtmenge der Öl-in-Wasser-Emulsionszusammensetzung, beträgt.

4. Öl-in-Wasser-Emulsionszusammensetzung gemäss irgendeinem der Ansprüche 1 bis 3, wobei der Gehalt der Komponente (B) 0,01 bis 20 Masse-%, bezogen auf die Gesamtmenge der Öl-in-Wasser-Emulsionszusammensetzung, beträgt.

5. Öl-in-Wasser-Emulsionszusammensetzung gemäss irgendeinem der Ansprüche 1 bis 4, wobei die Komponente (B) ein Sorbitan-Fettsäureester mit einem HLB von 1 bis 6 ist.

6. Öl-in-Wasser-Emulsionszusammensetzung gemäss irgendeinem der Ansprüche 1 bis 5, wobei der Gehalt der Komponente (C) 0,005 bis 8 Masse-%, bezogen auf die Gesamtmenge der Öl-in-Wasser-Emulsionszusammensetzung, beträgt.

7. Öl-in-Wasser-Emulsionszusammensetzung gemäss irgendeinem der Ansprüche 1 bis 6, wobei das Fettsäuretriglycerid drei Fettsäuregruppen mit jeweils 10 bis 22 Kohlenstoffatomen aufweist.

8. Öl-in-Wasser-Emulsionszusammensetzung gemäss irgendeinem der Ansprüche 1 bis 7, wobei das Fettsäuretriglycerid zumindest eines, ausgewählt aus der Gruppe bestehend aus Olivenöl, Macadamianussöl, Glyceryltri(caprylat/caprat), Glyceryltriisostearat, Glyceryltripalmitat, Glyceryltri-2-heptylundecanoat, Glyceryltribehenat, Glyceryltrimyristat, Glyceryltricocoat und Glyceryltrilaurat, ist.

9. Öl-in-Wasser-Emulsionszusammensetzung gemäss Anspruch 1, wobei die Komponenten (A), (B) und (C) wie folgt sind:

    (A) ein Öl, in dem ein Fettsäuretriglycerid mit drei Fettsäuregruppen, die jeweils 10 bis 22 Kohlenstoffatome aufweisen, in einer Menge von 80 bis 100 Masse-% in dem Öl enthalten ist und das Öl in einer Menge von 5 bis 50 Masse-%, bezogen auf die Gesamtmenge der Öl-in-Wasser-Emulsionszusammensetzung, enthalten ist,
    (B) ein Sorbitan-Fettsäureester der einen HLB von 3 bis 5,5 und eine verzweigte Fettsäuregruppe mit 15 bis 20 Kohlenstoffatomen aufweist, wobei der Sorbitan-Fettsäureester in einer Menge von 0,1 bis 12 Masse-%, bezogen auf die Gesamtmenge der Öl-in-Wasser-Emulsionszusammensetzung, enthalten ist und
    (C) 0,01 bis 3 Masse-% eines Mannosylerythritollipids, das zumindest eines, ausgewählt aus der Gruppe bestehend aus MEL-A, MEL-B, MEL-C and MEL-D, ist.

10. Öl-in-Wasser-Emulsionszusammensetzung gemäss irgendeinem der Ansprüche 1 bis 9, die im wesentlichen frei von einem Tensid mit einer Polyalkylenoxidgruppe ist.

11. Verwendung der Öl-in-Wasser-Emulsionszusammensetzung gemäss irgendeinem der Ansprüche 1 bis 10 für Kosmetika.

**Revendications**

1. Composition d’émulsion huile-dans-eau comprenant les composants (A) à (C) suivants, dans laquelle la HLB combinée des tensioactifs dans la composition est 5 à7:

(A) une huile dans laquelle un triglycéride d'acide gras ayant un ou plusieurs groupes d'acides gras ayant chacun 10 à 22 atomes de carbone est contenu en une quantité de 78 % en masse ou plus dans l'huile,
(B) un ester d'acide gras de sorbitane ayant une HLB de 6 ou moins, et
(C) un lipide de mannosyle érythritol.

**2.** Composition d'émulsion huile-dans-eau selon la revendication 1, dans laquelle le triglycéride d'acide gras ayant un ou plusieurs groupes d'acides gras ayant chacun 10 à 22 atomes de carbone dans le composant (A) est contenu en une quantité de 80 à 100 % en masse dans l'huile.

**3.** Composition d'émulsion huile-dans-eau selon la revendication 1 ou 2, dans laquelle la teneur en composant (A) est de 1 à 80 % en masse sur la base de la quantité totale de la composition d'émulsion huile-dans-eau.

**4.** Composition d'émulsion huile-dans-eau selon l'une quelconque des revendications 1 à 3, dans laquelle la teneur en composant (B) est de 0,01 à 20 % en masse sur la base de la quantité totale de la composition d'émulsion huile-dans-eau.

**5.** Composition d'émulsion huile-dans-eau selon l'une quelconque des revendications 1 à 4, dans laquelle le composant (B) est un ester d'acide gras de sorbitane ayant une HLB de 1 à 6.

**6.** Composition d'émulsion huile-dans-eau selon l'une quelconque des revendications 1 à 5, dans laquelle la teneur en composant (C) est de 0,005 à 8 % en masse sur la base de la quantité totale de la composition d'émulsion huile-dans-eau.

**7.** Composition d'émulsion huile-dans-eau selon l'une quelconque des revendications 1 à 6, dans laquelle le triglycéride d'acide gras a trois groupes d'acides gras ayant chacun 10 à 22 atomes de carbone.

**8.** Composition d'émulsion huile-dans-eau selon l'une quelconque des revendications 1 à 7, dans laquelle le triglycéride d'acide gras est l'un au moins sélectionné dans le groupe constitué d'huile d'olive, d'huile de noix de macadamia, de tri(caprylate/caprate) de glycéryle, de triisostéarate de glycéryle, de tripalmitate de glycéryle, de tri-2-heptylundécanoate de glycéryle, de tribéhénate de glycéryle, de trimyristate de glycéryle, de tricocoate de glycéryle, et de trilaurate de glycéryle.

**9.** Composition d'émulsion huile-dans-eau selon la revendication 1, dans laquelle les composants (A), (B), et (C) sont comme suit :

(A) une huile dans laquelle un triglycéride d'acide gras ayant trois groupes d'acides gras ayant chacun 10 à 22 atomes de carbone est contenu en une quantité de 80 à 100% en masse dans l'huile, l'huile est contenue en une quantité de 5 à 50 % en masse sur la base de la quantité totale de la composition d'émulsion huile-dans-eau,
(B) un ester d'acide gras de sorbitane ayant une HLB de 3 à 5,5 et ayant un groupe d'acide gras ramifié ayant 15 à 20 atomes de carbone, l'ester d'acide gras de sorbitane est contenu en une quantité de 0,1 à 12 % en masse sur la base de la quantité totale de la composition d'émulsion huile-dans-eau et
(C) 0,01 à 3 % en masse d'un lipide de mannosyle érythritol qui est au moins un sélectionné dans le groupe constitué de MEL-A, MEL-B, MEL-C et MEL-D.

**10.** Composition d'émulsion huile-dans-eau selon l'une quelconque des revendications 1 à 9, qui est substantiellement exempte d'un tensioactif ayant un groupe oxyde de polyalkylène.

**11.** Utilisation de la composition d'émulsion huile-dans-eau selon l'une quelconque des revendications 1 à 10, pour des cosmétiques.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007153858 A
- JP 11262653 A
- JP 11071261 A
- JP 2008106043 A
- JP 2009167159 A
- JP 2009149556 A
- JP 2009167157 A


### Non-patent literature cited in the description


- **GRIFFIN.** The HLB value by Griffin. *J. Soc. Cosm. Chem.,* 1954, vol. 5, 249-256